# EUROPEAN PATENT APPLICATION

(11) **EP 2 258 255 A1**
(43) Date of publication of application: **08.12.2010**
(21) Application number: 10164799.8
(22) Date of filing: 02.06.2010
(51) Int. Cl.: A61B 1/267, A61B 13/00

(54) **Tongue depressor and throat viewing apparatus**

(30) Priority: 02.06.2009 TW 098118275; 20.05.2010 EP 10163474
(71) Applicant: Chang, Hui-Yu, Taipei (TW)
(72) Inventor: Chang, Hui-Yu, Taipei (TW)
(74) Representative: Baldwin, Mark

(57) **Abstract**

A tongue depressor and throat viewing mechanism adapted to facilitate inspection of the larynx and a medical diagnosis of its condition, the mechanism includes a blade (10) insertable into the mouth to depress the tongue so as to cause the throat to expose the larynx; a power source (104) on the blade (10); a sleeve (26) on the blade (10); an insert (28) comprising a rear enlargement (30) and being adapted to insert into the sleeve (26) to be frictionally retained by the sleeve (26); a digital video camera (12) in a front end of the insert (28); a light emitting unit (14) in the front end of the insert (28); a transmitter (18) in the blade (10); and a switch (103) on the blade (10) for electrically connecting the power source (104) to the camera (12), the transmitter (18), and the light emitting unit (14).

## Description

### BACKGROUND OF THE INVENTION

### 1. Field of Invention

The invention relates to tongue depressors and more particularly to a tongue depressor and throat viewing mechanism for inserting into the tongue of a patient and taking a digital video image of the larynx in the throat for further examination.

### 2. Description of Related Art

Medical devices for examining the mouth including the larynx are well known. For example, a tongue depressor having a flat blade is used to depress the tongue of a patient in order to fully expose the larynx so that it can be inspected by a physician. Some tongue depressors have a camera provided behind or at the rear end of the flat blade. The camera may be attached to the tongue depressor by means of an adapter or a bracket. It is a natural response to bite the tongue depressor when the tongue depressor is placed in the open mouth of a patient (e.g., a baby or a child). As such, the view of the camera may be blocked so that it cannot obtain images of the larynx. Thus, the physician may take a prolonged period of time to obtain an image of the larynx. Furthermore, it may not be possible to obtain a complete image of the larynx by a rearwardly mounted camera. This can compromise the desired function of inspecting the larynx for diagnosis.

### SUMMARY OF THE INVENTION

The invention provides a tongue depressor and throat viewing mechanism having a forward camera which is proximate to the larynx after inserting a blade into the mouth so that the camera may yield a clear digital image of the larynx since, even if the patient bites the blade, no disruption or view blocking of the normal operation of the camera is possible.

The invention provides a tongue depressor and throat viewing apparatus adapted to facilitate inspection of the larynx of a subject, said apparatus comprising:
a blade having a front end and a rear end, the blade being configured for insertion into the mouth of the subject so that at least the front end depresses the subject's tongue for exposing the larynx;
a camera located at the front end and configured to provide image signals indicative of images captured by the camera; and
a light emitting unit for providing lighting to the camera.

The invention also includes a tongue depressor and throat viewing mechanism adapted to facilitate inspection of the larynx of a subject and a medical diagnosis of its condition, the tongue depressor and throat viewing mechanism comprising a flat blade insertable into the mouth of the subject to depress the tongue so as to cause the throat to expose the larynx; a power source disposed on the blade; a sleeve disposed on the blade; an elongated insert comprising a rear enlargement and being adapted to insert into the sleeve to be frictionally retained by the sleeve when the enlargement is stopped at a rear end of the sleeve; a miniature, digital video camera disposed in a front end of the insert ; a light emitting unit disposed in the front end of the insert; a transmitter disposed in the blade; and a switch disposed on the blade and being proximate to the power source, the switch being electrically connecting the power source to each of the camera, the transmitter, and the light emitting unit, wherein in response to inserting the blade into the mouth, activating the light emitting unit to project a light beam to illuminate the exposed larynx, activating the transmitter, and activating the camera by manually activating the switch, an image of the larynx is yielded by the camera and the image can be sent by the transmitter for exhibit.

The invention also includes a tongue depressor and throat viewing apparatus adapted to facilitate inspection of the larynx of a subject, said apparatus comprising an elongate member insertable into the mouth of the subject to depress the tongue so as to cause the throat to expose the larynx and an image capturing device disposed at a leading end region of said elongate member such that if the subject closes its mouth the larynx can still be viewed by said image capturing device.

The apparatus may be provided with a light emitting unit provided at said leading end region.

At least one of said light emitting unit and image capturing device may be disposed within said elongate member.

The apparatus may be provided with a transmitter for transmitting image data representative of images captured by said image capturing device to a remote receiver.

The elongate member may be provided with a housing for said image capturing device and said image capturing device may be releasably push-fit engageable in said housing.

The apparatus may comprise circuitry for at least one of said image capturing device and/or said light-emitting unit, said circuitry being disposed within said elongate member.

The above features and advantages of the invention will become apparent from the following detailed description taken with the accompanying drawings.

### BRIEF DESCRIPTION OF THE DRAWINGS

FIG. 1 is a perspective view of a tongue depressor and throat viewing mechanism according to a first preferred embodiment of the invention;
FIG. 1A is a greatly enlarged view of a front end of FIG. 1;
FIG. 2 is a cross-sectional view of FIG. 1;
FIG. 3 is a perspective view of a tongue depressor and throat viewing mechanism according to a second preferred embodiment of the invention;
FIG. 4 is a perspective view of a tongue depressor and throat viewing mechanism according to a third preferred embodiment of the invention;
FIG. 4A is a greatly enlarged view of a front end of FIG. 4;
FIG. 5 is a cross-sectional view of FIG. 4;
FIG. 6 is a perspective view of a tongue depressor and throat viewing mechanism according to a fourth preferred embodiment of the invention;
FIG. 7 is an exploded view of a tongue depressor and throat viewing mechanism according to a fifth preferred embodiment of the invention;
FIG. 8 is a block diagram of the tongue depressor and throat viewing mechanism, a channel indicator, and a computer system in cooperation therewith;
FIG. 9 is a perspective view of a tongue depressor and throat viewing mechanism according to a sixth preferred embodiment of the invention; and
FIG. 10 is a perspective view of a tongue depressor and throat viewing mechanism according to a seventh preferred embodiment of the invention.

### DETAILED DESCRIPTION OF THE INVENTION

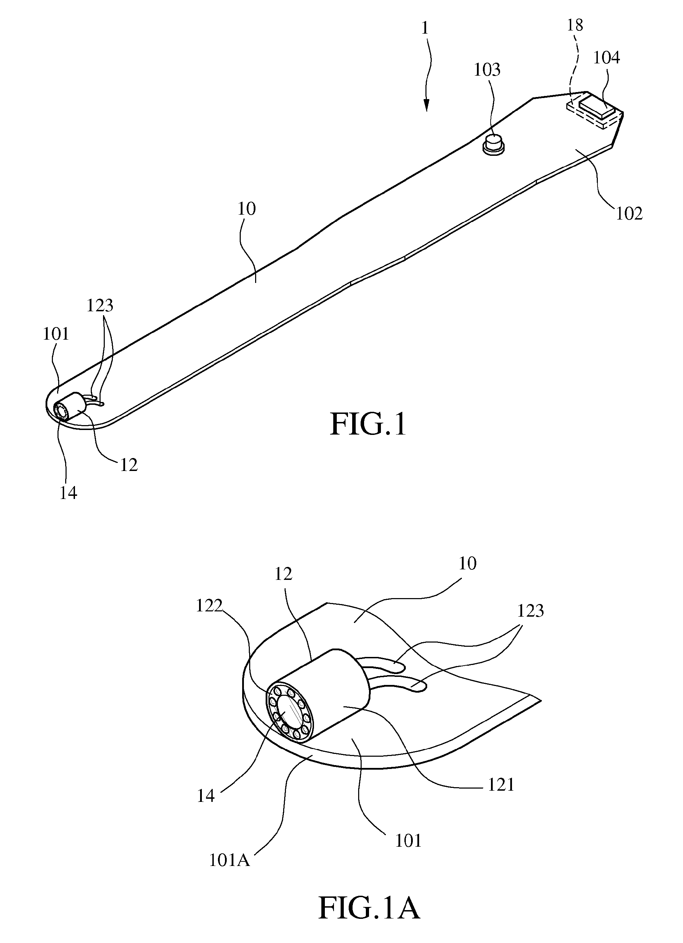

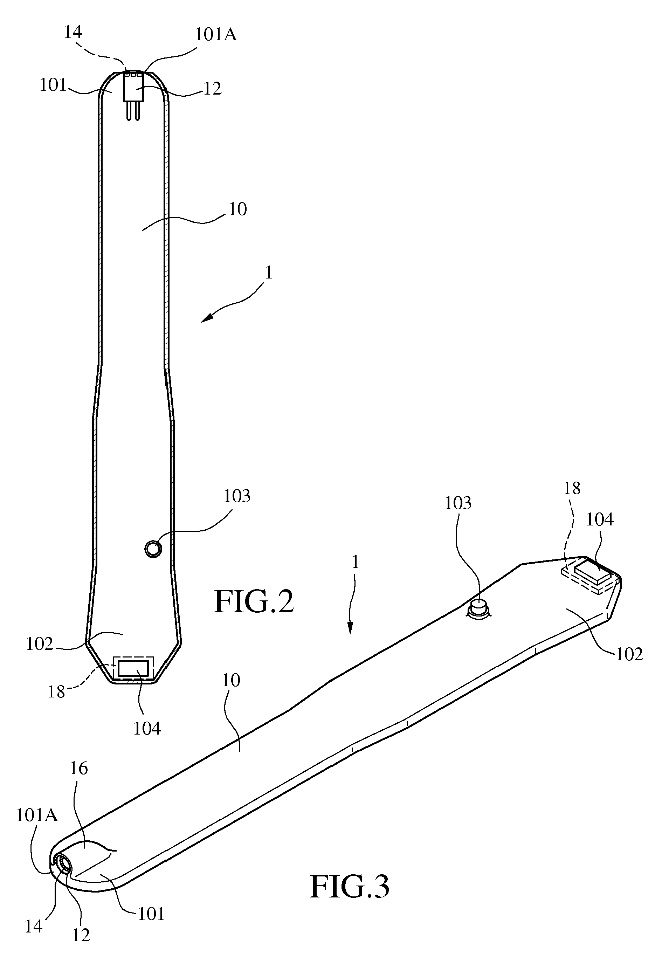

Referring to FIGS. 1, 1A and 2, a tongue depressor and throat viewing mechanism 1 in accordance with a first preferred embodiment of the invention comprises the following components as discussed in detail below.

A flat blade 10 has a front end 101 and a rear end 102. A miniature, digital video camera 12 is provided in a cylinder 121 which is mounted on top of the front end 101. A lens 122 is provided on an opening of the cylinder 121 above a curved edge 101A on the front end 101. A light emitting unit 14 is provided in the camera 12. Power and cable 123 are interconnected the camera 12 and a printed circuit board (PCB) (not shown) is within the blade 10. A power source (e.g., battery) 104 is provided on a top position proximate to the rear end 102. A switch (e.g., push button switch) 103 is provided between the power source 104 and a center of the blade 10. The switch 103 is electrically interconnected with the power source 104 and the PCB for permitting a power supply from the power source 104 to the PCB or not. Moreover, power can be supplied from the PCB to the camera 12 and the light emitting unit 14. Preferably, the light emitting unit 14 comprises a plurality of light-emitting diodes (LEDs) 14.

In use, a physician may press the switch 103 to activate both the camera 12 and the light emitting unit 14 which thus can project a light beam. Next, the blade 10 is inserted into the mouth of a patient (e.g., baby or child) to press down the tongue for inducing a gag reflex. Naturally, the throat may dilate to fully expose the larynx for viewing. The camera 12, provided on the front end 101 of the blade 10, is proximate to the larynx. Thus, the camera 12 may yield a clear digital image of the larynx which is being illuminated by the light emitting unit 14 since, even if the patient bites the blade 10, no disruption or view blocking of the normal operation of the camera 12 is possible. The image of the larynx is then wirelessly sent by a transmitter 18 to a display (not shown) of a computer (not shown) in a magnified scale. Note that details of the wireless transmission will be discussed later. Therefore, the physician may clearly see the larynx from the display and a correct diagnosis thus can be made.

Referring to FIG. 3, a tongue depressor and throat viewing mechanism in accordance with a second preferred embodiment of the invention is characterized below. The cylinder 16 is integrally formed with the top of the front end 101. Both the camera 12 and the light emitting unit 14 are provided in the cylinder 16.

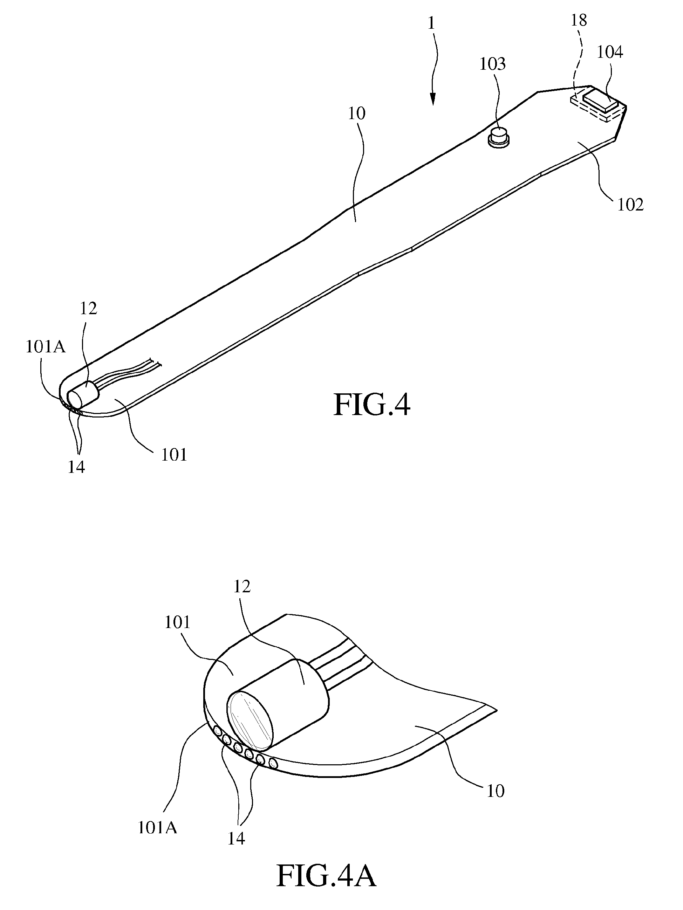

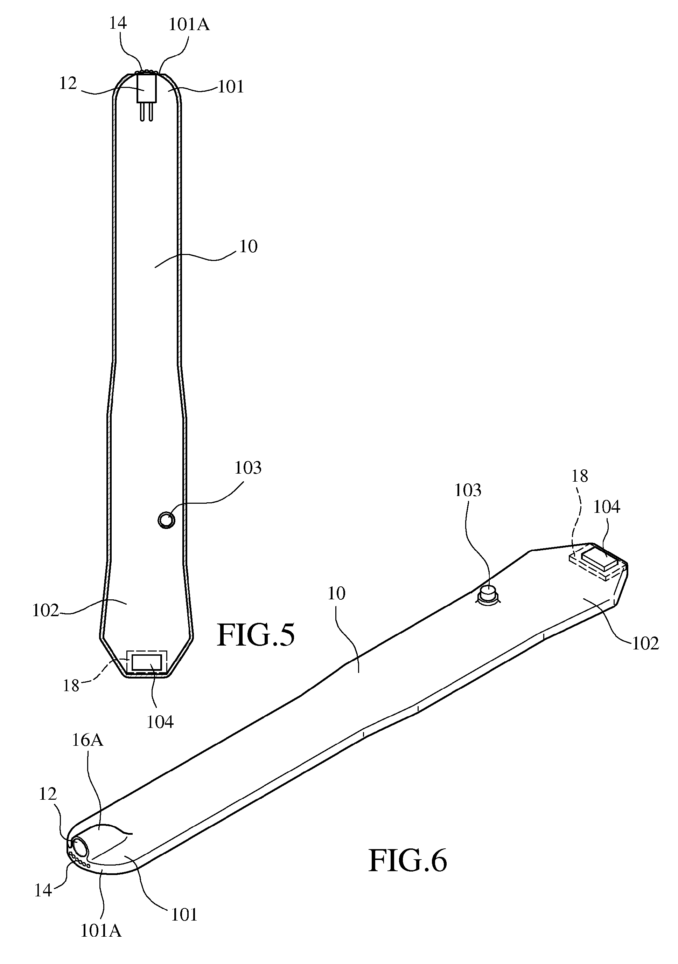

Referring to FIGS. 4, 4A and 5, a tongue depressor and throat viewing mechanism in accordance with a third preferred embodiment of the invention is characterized below. The light emitting unit 14 comprises a plurality of LEDs 14 which are provided on the curved edge 101 A on the front end 101.

Referring to FIG. 6, a tongue depressor and throat viewing mechanism in accordance with a fourth preferred embodiment of the invention is characterized below. The cylinder 16A is integrally formed with the top of the front end 101. The camera 12 is provided in the cylinder 16A. The light emitting unit 14 comprises a plurality of LEDs 14 which are provided on the curved edge 101A on the front end 101.

The cylinder 16A is integrally formed with the top of the front end 101. The camera 12 is provided in the cylinder 16A. The light emitting unit 14 comprises a plurality of LEDs 14 which are provided on the curved edge 101A on the front end 101. A transmitter 18 is provided on top of the rear end 102. The transmitter 18 is electrically connected to the power source 104 and is in signal communication with the camera 12.

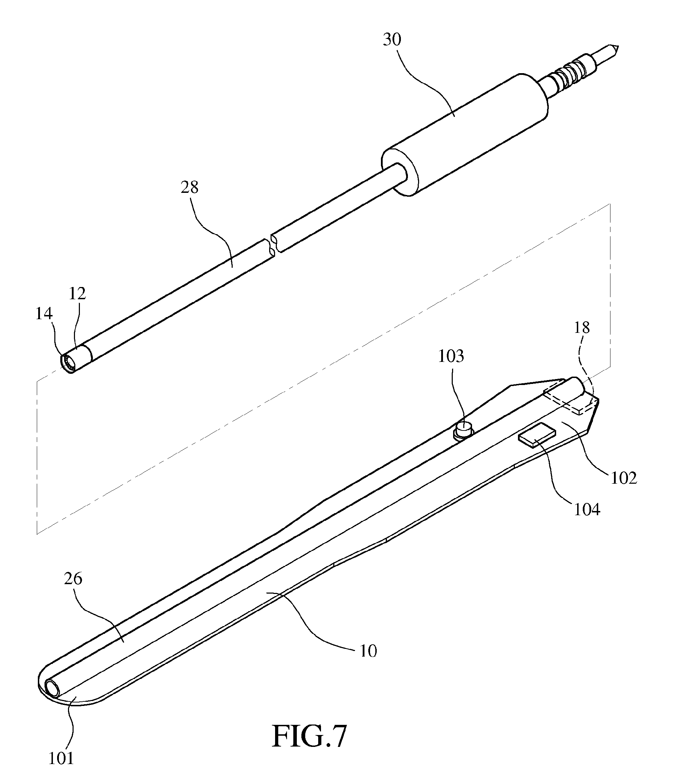

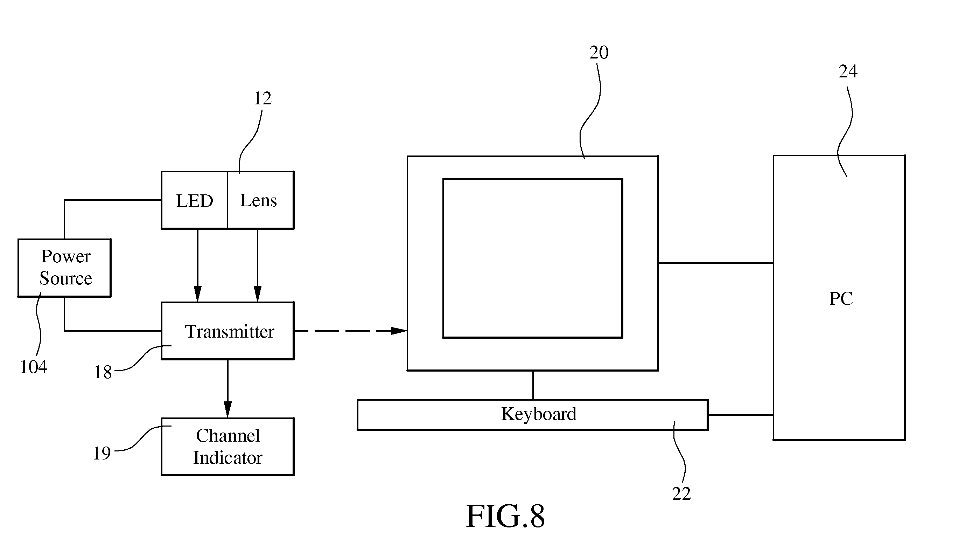

Referring to FIGS. 7 and 8, a tongue depressor and throat viewing mechanism in accordance with a fifth preferred embodiment of the invention is characterized below. A lengthwise sleeve 26 of circular section is provided on top of the blade 10. A cylindrical insert 28 has a front end provided with a camera 12 and a light emitting unit 14 and a rear end provided with an enlargement 30. The insert 28 can be inserted into the sleeve 26 until the enlargement 30 contacts the sleeve 26. At this position, the insert 28 is frictionally retained in the sleeve 26 and the camera 12 is disposed at the front end of the sleeve 26 (i.e., on top of the front end 101). Switch 103, transmitter 18, and power source 104 are provided proximate to the rear end 102. This embodiment facilitates a replacement of the camera 12 if such need arises.

Thus, image signals can be sent from the camera 12 to the transmitter 18 which in turn wirelessly transmits same to a personal computer (PC) 24 and transmits same to a channel indicator 19 by wire. The channel indicator 19 is provided proximate to where the physician performs the examination so that the physician may see the operating channel of the transmitter 18. Moreover, the PC 24 is equipped with a display 20 for showing the magnified examination image of the larynx, and a keyboard 22.

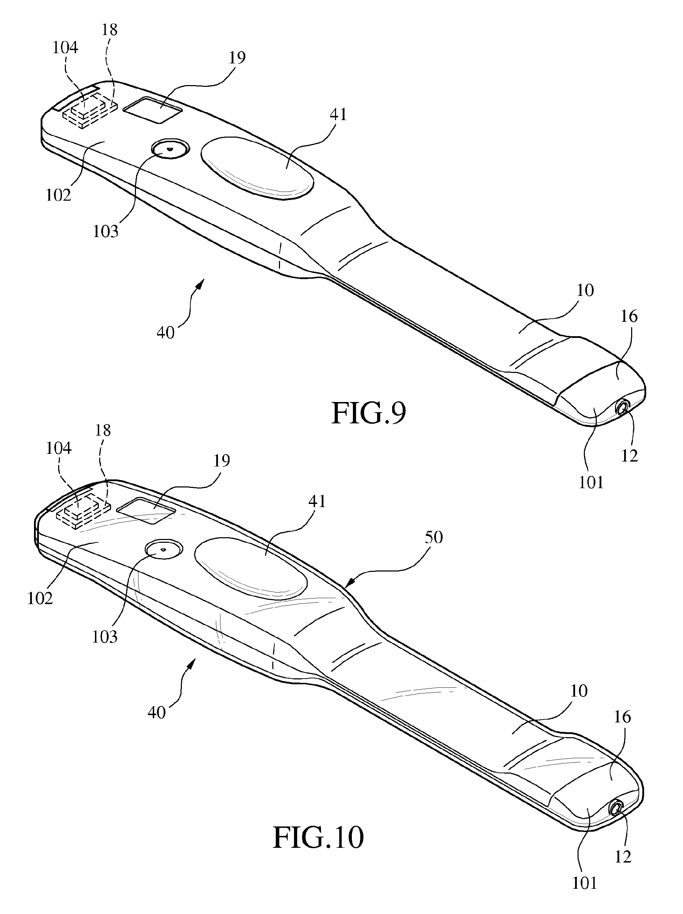

Referring to FIG. 9, a tongue depressor and throat viewing mechanism in accordance with a sixth preferred embodiment of the invention is characterized below. A flexible sheath 40 is wrapped around the blade 10. The sheath 40 has an oval depression 41 for ease of manual pushing of the blade 10 into the tongue. Also, the switch 103 and the channel indicator 19 are provided proximate to the rear end 102. The camera 12 is provided in the cylinder 16 which is integrally formed on top of the front end 101.

Referring to FIG. 10, a tongue depressor and throat viewing mechanism in accordance with a seventh preferred embodiment of the invention is characterized below. An additional transparent, flexible, disposable enclosure 50 made of plastic is put on the sheath 40. After a single use, the enclosure 50 can be discarded for hygienic purposes.

The embodiments provide the following advantages. In use, after inserting the blade into the mouth of a patient to press down the tongue, the throat may dilate to fully expose the larynx for viewing. The camera is relatively small (e.g., about or less than 5 mm in diameter) and so can be disposed on a front end of the tongue depressor in the throat proximate to the larynx so that the camera may yield a clear digital image of the larynx. Thus, even if the patient bites the blade, no disruption or view blocking of the normal operation of the camera is possible. Moreover, the image of the larynx can be sent to a display of a computer for showing in a magnified scale by wireless transmission. Thus, a correct diagnosis can be made.

The embodiments provide a tongue depressor and throat viewing apparatus adapted to facilitate inspection of the larynx of a subject, such as a person. The apparatus has a blade 10 with a front end 101 and a rear end 102. Thee blade is configured to be inserted into the mouth of the subject so that at least the front end depresses the subject's tongue for exposing the larynx. A camera 12 located at the front end 101 is used to take images of the larynx and the surrounding area. A light emitting unit is used to provide lighting to the camera. The camera can send images to a transmitter 18 which is configured for transmitting the image signals for display. The transmitter can be located at the rear end of the blade, or may be a separate unit. The apparatus also has a switch for electrically connecting the light emitting unit and the camera to a power source. The switch is manually operated when needed. The power source can be a battery of a small size so it can be attached to the rear end of the blade.

The camera can be mounted on a mount located in the front end. The light emitting unit can have one or more light emitting diodes located on a front-curved edge of the blade. Alternatively, the light emitting unit may comprises a plurality of light emitting diodes mounted around the circumference of the camera lens. In one embodiment, a flexible sheath 40 is provided around the blade to facilitate pushing of the blade into the mouth. Another embodiment is provided with a disposable wrap for wrapping around the blade, with or without the flexible sheath. The disposable wrap 50 can be made of transparent and flexible material. In yet another embodiment, an elongate insert 28 having a first end is used for mounting the camera, and a lengthways extending sleeve 26 is located on the blade. The elongate insert is dimensioned for inserting through the sleeve so that the camera can be located at the front end of the blade. Preferably, the elongate insert 28 has an opposing second end with an enlarged portion for limiting the insertion of the elongate insert into the sleeve. The transmitter may be configured to provide an electrical signal to a channel indicator 19 for indicating an operating channel of the transmitter.

While the invention has been described in terms of preferred embodiments, those skilled in the art will recognize that the invention can be practiced with modifications within the scope of the appended claims.

## Claims

**1.** A tongue depressor and throat viewing apparatus adapted to facilitate inspection of the larynx of a subject, said apparatus comprising:
a blade (10) having a front end (101) and a rear end (102), the blade being configured for insertion into the mouth of the subject so that at least the front end depresses the subject's tongue for exposing the larynx;
a camera (12) located at the front end and configured to provide image signals indicative of images captured by the camera; and
a light emitting unit (14) for providing lighting to the camera.

**2.** An apparatus according to claim 1, further comprising:
a transmitter (18) configured for receiving the image signals and for transmitting the image signals for display.

**3.** An apparatus according to claim 2, wherein the transmitter is located at the rear end of the blade (10).

**4.** An apparatus according to claim 2 or 3, wherein the transmitter is configured to provide an electrical signal to a channel indicator (19) for indicating an operating channel of the transmitter.

**5.** An apparatus according to claim 2, 3 or 4, further comprising:
a power source (104) located near the rear end (102); and
a switch (103) located near the rear end and configured for electrically connecting the light emitting unit (14) and the camera with the power source.

**6.** An apparatus according to claim 5, wherein the switch is also configured for electrically connecting the transmitter with the power source.

**7.** An apparatus according to any one of the preceding claims, further comprising:
an elongate insert (28) having a first end for mounting the camera; and
a lengthways extending sleeve (26) disposed on the blade, the elongate insert being configured for insertion into the sleeve such that the camera is located at the front end of the blade.

**8.** An apparatus according to claim 7, wherein the elongate insert (28) has a second end provided with an enlarged portion for limiting insertion of the elongate insert into the sleeve (26).

**9.** An apparatus according to any one of claims 1 to 6, further comprising a mount disposed at the front end (101) for mounting the camera and the light emitting unit.

**10.** An apparatus according to any one of the preceding claims, wherein the camera comprises a camera lens and the light emitting unit (14) comprises a plurality of light-emitting diodes surrounding the lens.

**10.** An apparatus according to any one of the preceding claims, wherein the front end of the blade comprises a curved edge (101A) and the light emitting unit (14) comprises one or more light-emitting diodes disposed on the curved edge.

**12.** An apparatus according to any one of the preceding claims, further comprising a flexible sheath (40) around the blade for facilitating insertion of the blade into the mouth.

**13.** An apparatus according to any one of the preceding claims, further comprising a disposable wrap (50) for wrapping around the blade.

**14.** An apparatus according to claim 13, wherein the disposable wrap (50) is made of transparent and flexible material.

**15.** An apparatus according to any one of the preceding claims, wherein the camera has a diameter substantially equal to or smaller than 5mm.
